# EUROPEAN PATENT SPECIFICATION

(11) **EP 1 827 508 B1**
(45) Date of publication and mention of the grant of the patent: **19.08.2009**
(21) Application number: 05824278.5
(22) Date of filing: 12.12.2005
(51) Int. Cl.: A61K 49/18

(54) **TARGETING AGENTS FOR MOLECULAR IMAGING**
TARGETINGMITTEL FÜR MOLEKULARE BILDERZEUGUNG
AGENTS DE CIBLAGE POUR UNE IMAGERIE MOLECULAIRE

(30) Priority: 17.12.2004 EP 04106696
(43) Date of publication of application: 05.09.2007
(73) Proprietor: Koninklijke Philips Electronics N.V., 5621 BA Eindhoven (NL); Philips Intellectual Property & Standards GmbH, 20099 Hamburg (DE)
(72) Inventor: HUMMEL, Helga, NL-5656 AA Eindhoven (NL); WEILER, Volker, U., NL-5656 AA Eindhoven (NL)
(74) Representative: Schouten, Marcus Maria
(86) International application number: PCT/IB2005/054187
(87) International publication number: WO 2006/064453

(56) References cited:
- WO-A-20/05046733
- WO-A-20/05107818
- US-B1- 6 333 110

## Description

The present invention pertains to targeting contrast agents and targeting therapeutic agents, methods for their production and use thereof.

Known imaging techniques with tremendous importance in medical diagnostics are positron emission tomography (PET), computed tomography (CT), magnetic resonance imaging (MRI), single photon computed tomography (SPECT) and ultrasound (US). Although today's imaging technologies are well developed they rely mostly on nonspecific, macroscopic, physical, physiological, or metabolic changes that differentiate pathological from normal tissue.

Targeting molecular imaging (MI) has the potential to reach a new dimension in medical diagnostics. The term "targeting" is related to the selective and highly specific binding of a natural or synthetic ligand (binder) to a molecule of interest (molecular target) in vitro or in vivo.

MI is a rapidly emerging biomedical research discipline that may be defined as the visual representation, characterization and quantification of biological processes at the cellular and sub-cellular levels within intact living organisms. It is a novel multidisciplinary field, in which the images produced reflect cellular and molecular pathways and in vivo mechanism of disease present within the context of physiologically authentic environments rather than identify molecular events responsible for disease.

Several different contrast-enhancing agents are known today and their unspecific or nontargeting forms are already in clinical routine. Some examples listed below are reported in literature.

For example, Gd-complexes could be used as contrast agents for MRI according to "Contrast Agents I" by W. Krause (Springer Verlag 2002, page one and following pages). Furthermore, superparamagnetic particles are another example of contrast-enhancing units, which could also be used as contrast agents for MRI (Textbook of Contrast Media, Superparamagnetic Oxides, Dawson, Cosgrove and Grainger Isis Medical Media Ltd, 1999, page 373 and following pages). As describe in Contrast Agent II by W. Krause (Springer Verlag 2002, page 73 and following pages), gas-filled microbubbles could be used in a similar way as contrast agents for ultrasound. Moreover "Contrast Agents II" by W.Krause (Springer Verlag, 2002, page 151 and following pages) reports the use of iodinated liposomes or fatty acids as contrast agents for X-Ray imaging.

Contrast-enhancing agents that can be used in functional imaging are mainly developed for PET and SPECT.

One example of these contrast agents are ¹⁸F-labelled molecules such as desoxyglucose (Beuthien-Baumann B, et al., (2000), Carbohydr. Res., 327, 107). The use of these labeled molecules as contrast agents for PET is described in "Contrast Agents II" by W. Krause (Springer Verlag, 2002, page 201 and following pages). But they only accumulate in tumor tissue without any prior specific cell interaction. Further on, ⁹⁹Tc-labelled molecules like antibodies or peptides could be used as targeting contrast agents for SPECT (Verbruggen A.M., Nosco D.L., Van Nerom C.G. et al., 99mTc-L,L-ethylenedicysteine: a renal imaging agent, Nucl. Med. 1992,33,551-557), but the labeling of such complex molecules is very difficult and costly.

The same can be said about several other ligands already existing for use in PET/SPECT, e.g. L-DOPA (dopamine receptor, Parkinson) (Luxen A., Guillaume M, Melega WP, Pike VW, Solin O, Wagner R, (1992) Int. J. Rad. Appl. Instrum. B 19, 149); Serotonin analogue (serotonin receptor) (Dyck CH, et al., 2000, J. Nucl. Med., 41, 234); Somatostatin analogue (somatostatin, oncology) (Maecke, H.R. et al., Eur. J. Nucl. Med. Mol. Imaging, 2004, Mar. 17), Peptide for integrin receptors (angiogenesis) (Wicklinde, S. A. et al., Cancer Res., 2003 Sep. 15, 63(18), 5838-43; Wicklinde, S. A. et al., Circulation 2003 Nov. 4, 108, (18), 2270-4).

WO 01/09193 A1 and US 6,437,095 B1 claim chimeric polypeptides, a method for production by directed association of peptides and disulfide bond formation and uses thereof for multimeric pharmaceutical agents (therapeutics). Electrostatic interaction promotes the directed association of two synthetic peptides and subsequent disulfide bond formation as described in S.A. Richter et al., Protein Engineering, 2001, vol. 14, no. 10, pp 775-783.

However, the identification of specific molecular events responsible for disease is of increasing importance in medicine. Targeting agents which are equipped with molecular recognition mechanisms to enrich contrast-enhancing materials specifically in certain tissues in vivo or in vitro and allow insights into molecular pathology are therefore essential to diagnosis and future therapy as well.

Thus, it is an object of the present invention to provide a new generation of improved contrast agents which allow an early diagnosis with high sensitivity and specificity as well as differential diagnosis and to supply methods for producing said improved contrast agents which are less costly and time consuming. Here is would also be advantageous to provide production processes and targeting agents produced thereby, which can be easily adapted to actually occurring problems which have to be solved in short time and with low effort concerning cost and man power. Apart from their potential for imaging diagnostics, targeting contrast agents will also play a crucial role in the development of new therapeutics. Such targeting contrast agents are still not available at the moment.

The object of the present invention is advantageously solved through the present invention as described below and additionally through the claims and examples. Preferred variants are described in the Figures and used for explanation of the invention but are not limiting.

The present invention pertains to one advantageous variant of a method for the production of a targeting contrast agent or targeting therapeutic agent, which method comprises the steps of:
a) providing a core;
b) adding a shell to the core;
c) modifying the shell through attaching at least a first polypeptide comprising at least one cysteine;
d) providing a ligand bearing a second complementary polypeptide comprising at least one cysteine; and
e) linking at least one ligand to the shell via a linking unit which is formed through electrostatic association between said first polypeptide and said second complementary polypeptide followed by at least one disulfide bond formation between said cysteines.

In a further embodiment of said method more than one shell can be added to the core in step b). Or in other words, the outer shell can be separated from the core by one to several inner shells. In preferred embodiments of the present invention the core can be separated from the outer shell by 1 to 100 inner shell(s), more preferred by 1 to 50 inner shell(s). Thereby the shell(s) can comprise a monolayer or a polylayer. Each of these shells (which can comprise a monolayer or a polylayer of an appropriated material in preferred embodiments of the present invention) has a thickness of about 0.5nm to 100nm. In a preferred embodiment of the present invention, each shell has a thickness of about 05nm to 500nm. Furthermore, each shell or even several shells can comprise the same material or different materials.

In a further variant of the present invention the shell(s) can cover the core at least partially. This is preferably the case when e.g. an organic polymer (e.g. polyethylenglycol/ PEG, polyvinylalcohol/PVA, polyamide, polyacrylat, polyurea), an organic polymer with functional end groups (e.g. 1.2-Distearoyl-sn-Glycero-3-Phosphoethanolamine-N-[Carboxy(polyethylene glycol)2000] ammonium salt), a biopolymer (e.g. polysaccharide such as dextran, xylan, glycogen, pectin, cellulose or polypeptide such as collagen, globulin), cysteine or a peptide with high cysteine content or a phospholipid is used as shell(s). In the sense of the present invention adding a shell to the core means completely surrounding the core, covering only some distinct areas and preferably all ranges between these situations.

For a special application it might be suitable not to add one or more shell(s) to the core. Thus the present invention also pertains to a variant method for the production of a targeting contrast agent, the method comprising the steps of:
a) providing a core;
b) modifying the core through attaching at least a first polypeptide comprising at least one cysteine;
c) providing a ligand bearing a second complementary polypeptide comprising at least one cysteine; and
d) linking at least one ligand to the core via a linking unit which is formed through electrostatic association between said first polypeptide and said second complementary polypeptide followed by a disulfide bond formation between said cysteines.

Advantageous variations of present methods for the production of a targeting contrast agent or targeting therapeutic agents are described through the depending claims asattached.

In detail the present invention has several particular advantageous variations described as set out below:

The "core", means material suitable as a contrast-enhancing part and/ or the therapeutic part of the present targeting contrast agent. Said core is covalently and ionically bonded to the ligand, because of the particular structure of the polypeptides used as a linking unit.

In the sense of the present invention the expression "ligand" can be used as a synonym for binder or preferably for biologically active ligand.

The expression "linking unit" means the association of at least two polypeptides during the production process.

Advantageously, the "first polypeptide" comprises 1 to 3 cysteines and 4 to 12 basic amino acids selected from the group consisting of arginine, lysine and ornithine or 4 to 12 acidic amino acids selected from the group consisting of glutamate and aspartate and the "second complementary polypeptide" comprises 1 to 3 cysteines and 4 to 12 acidic amino acids selected from the group consisting of glutamate and aspartate or 4 to 12 basic amino acids selected from the group consisting of arginine, lysine and ornithine, wherein the group of basic amino acids selected for the first and second polypeptides are different.

Consequently, "complementary sequences" means either basic or acidic amino acids or any other amino acids which interact contrary to each other with respect to their electrostatic charging.

The "shell(s)" means material that can allow a good dispersion of the targeting contrast agent, can decrease its toxicity or can prevent adverse effects, depending on which material is used as ashell. If nanoparticles are used as the core, the use of an appropriated shell (e.g. a shell of ZnS) can reduce the number of surface defects of the nanoparticles. These defects considerably reduce the contrast generated by the nanoparticles. Therefore, reducing the number of defects leads to better targeting contrast-enhancing agents.

The ionic and covalent bond between said polypeptides can be generated under mild reaction condition in aqueous media, so that preferably the ligand keeps its full biological activity. This is possible because the electrostatic interactions between the two complementary polypeptides allow the formation of the disulfide bond under particularly mild conditions and because both the bonding unit and the contrast-enhancing cores (or eventually the core/shell(s) assemblies) are water-soluble.

"Mild conditions" preferably means art-known conditions under which the ligand will retain its activity and specificity, respectively, e.g. condition in aqueous solution or blood- or serum-such as solutions, physiological pH values at room temperature.

The "linking" is performed site-specifically at the cysteines of said polypeptides attached to the core and/or shell(s) or ligand, respectively. Because fixing said polypeptides to the ligand (or core/shell(s)) is possible in a directly controlled and highly selective way the catalytic or regulatory center or the recognition sites for specific binding of the ligand will retain its natural activity or avoids the deactivation of the ligand.

The expression "modifying the core/ shell(s) through attaching" has to be understood in the same way as the fixing of said polypeptides to the ligand.

The shell(s) can comprise further components", e.g. proteins which enable the passage of the complete targeting agents through e.g. cell membranes (e.g. the HIV-tat peptide, etc) or to increase the biocompatibility or decrease the toxicity.

The methods disclosed in this invention are potentially applicable to any ligand and any contrast-enhancing material or therapeutic material, providing a very versatile and easily adaptable system for the preparation of any kind of targeting contrast agent or targeting therapeutic agent.

The present invention further pertains to targeting contrast agents and targeting therapeutic agents and the use thereof.

The present targeting contrast agent has the following characteristics which describe the invention but are not limiting:

Depending on the contrast-enhancing material the targeting contrast agent can be applied in different imaging procedures such as MRI, US, SPECT, CT, PET, optical imaging or multimodalit approaches like PET/CT.

The targeting contrast agent comprising a contrast-enhancing core (e.g. magnetic nanoparticle) or therapeutic core that can be covered by one ore more shells to improve stability and /or biocompatibility and/or to reduce toxicity in vivo (e.g. PEG shell).

If nanoparticles are used as the core, the size of these particles may vary from about 1nm to 200nm. In preferred embodiments of the present invention, the size of particles may vary from 1nm to 100nm.

If polymers are used as shell(s), the molecular weight of these polymers may vary from 200g/mol to 200000g/mol. In preferred embodiments of the present invention, the molecular weight of these polymers may vary from 200g/mol to 100000g/mol.

The targeting contrast agent comprises a targeting ligand.

A most preferred variant of a present targeting contrast agent comprises a core, at least one linking unit and at least one ligand.

Moreover, the present invention pertains to advantageous specifications of targeting contrast agents or targeting therapeutic agents comprising a core, at least one shell, at least one linking unit and at least one ligand.

Object of the present invention are further targeting contrast agents or targeting therapeutic agents produced by any one of the described or claimed methods.

The present targeting contrast agents or targeting therapeutic agents are suitable for use in diagnosis or therapy, preferably in targeting molecular imaging.

Targeting contrast agents for use in CT, MRI, PET, SPECT or US are also included in the present invention.

Moreover, the use of the present targeting contrast agents or targeting therapeutic agents for the production of compounds suitable in diagnosis or therapy are object of the present invention as well as the use of the targeting contrast agents or targeting therapeutic agents for the production of compounds suitable for targeting molecular imaging. Additionally, the present invention pertains to the use of the present targeting contrast agents for the production of compounds suitable in CT, MRI, PET, SPECT or US.

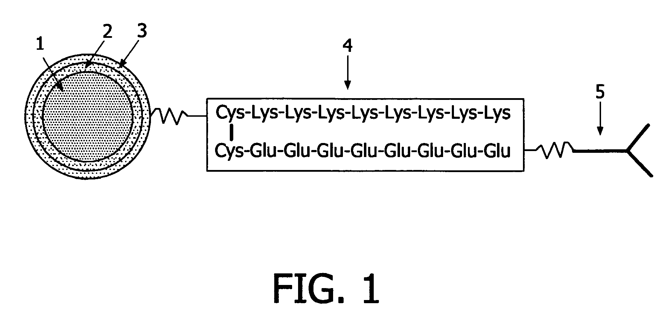

A most preferred variant of the present targeting contrast agent is described schematically in Figure 1.

### Description of Figures in detail:

### Figure 1:

- Core (1): e.g. (not limited to this) contrast-enhancing material; or therapeutical material for:
   - MRI: e.g. (not limited to these) ferro-, antiferro-, ferrimagnetic or superparamagnetic material such as iron (Fe), iron oxide γ-Fe2O3 or Fe₃O₄ or ferrit with spinell structure MFe₂O₄ (M = Mn, Co, Ni, Cu, Zn, Cd) or ferrit with granat structure M₃Fe₅0₁₂ (M = Y, La, Ce, Pr, Nd, Sm, Eu, Gd, Tb, Dy, Ho, Er, Tm, Yb, Lu) or ferrit with a magnetoplumbit structure MFe₁₂O₁₉ (M = Ca, Sr, Ba, Zn) or other hexagonal ferrit structures like e.g. Ba₂M₂Fe₁₂O₂₂ (M = Mn, Fe, Co, Ni, Zn, Mg); in all cases the core can be doped with additional 0.01 to 5.00 mol-% of Mn, Co, Ni, Cu, Znor F.
      Paramagnetic ion (e.g. lanthanide, manganese, iron, copper) based contrast-enhancing units e.g. Gadolinium chelates like Gd(DTPA), Gd(BMA-DTPA), Gd(DOTA), Gd(D03A); oligomeric structures; macromolecular structures such as Albumin Gd(DTPA)20-35, Dextran Gd(DTPA), Gd(DTPA)-24-cascade polymer, polylysine-Gd(DTPA), MPEG polylysine-Gd(DTPA); dendrimeric structures of lanthanide based contrast-enhancing units; Manganese-based contrast-enhancing units such as Mn(DPDP), Mn(EDTA-MEA), poly-Mn(EED-EEA), and polymeric structures; liposomes as carriers of paramagnetic ions e.g. liposomal Gd(DTPA); non-proton imaging agents;
   - Optical: e.g. (not limited to these) luminescent material such as nanophosphores (e.g. rare earth doped YPO₄ or LaPO₄ or semiconducting nanocrystals (so called quantum dots; e.g. CdS, CdSe, ZnS/CdSe, ZnS/CdS); carbocyanine dyes; tetrapyrrole-based dyes (porphyrins, chlorins, phthalocyanines and related structures); deltaaminolevulinic acid; fluorescent lanthanide chelates; fluorescein or 5- aminofluorescein or fluorescein-isothiocyanate (FITC) or other fluorescein-related fluorophors such as Oregon Green, naphthofluorescein;
   - US: e.g. (not limited to these) shell (e.g. protein, lipid, surfactant or polymer) encapsulated gas (e.g. air, perfluorpropane, dodecafluorcarbon, sulphur hexafluride, perfluorcarbon) bubbles (like Optison from Amersham, Levovist from Schering); shell (e.g. protein, lipid, surfactant or polymer) encapsulated droplets; nanoparticles (e.g. platinum, gold, tantalum);
   - X-Ray: e.g. (not limited to these) iodinated contrast-enhancing units like e.g. ionic and non-ionic derivatives of 2,4,6-tri-iodobenzene; barium sulfate-based contrast-enhancing units; metal ion chelates such as e.g. gadolinium based compounds; boron clusters with high proportion of iodine; polymers such as iodinated polysaccharides, polymeric triiodobenzenes; particles from iodinated compounds displaying low water solubility; liposomes containing iodinated compounds; iodinated lipids such as triglycerides, fatty acids;
   - PET: e.g. (not limited to these) ¹¹C, ¹³N, ¹⁵O, ^{66/8}Ga, ⁶⁰Cu ⁵²Fe, ⁵⁵CO, ^{61/2/4}Cu, ^{62/3}Zn, ^{70/1/4}As, ^{75/6}Br ⁸²Rb, ⁸⁶Y, ⁸⁹Zr, ¹¹⁰In, ^{120/4}I,¹²²Xe and ¹⁸F based tracers, such as e.g. ¹⁸F-FDG (glucose metabolism); ¹¹C-Methionine, ¹¹C-Tyrosine, ¹⁸F-FMT, ¹⁸F-FMT or ¹⁸F-FET (amino acids); ¹⁸F-FMISO, ⁶⁴Cu-ATSM (hypoxia); ¹⁸F-FLT, ¹¹C-Thymidine, ¹⁸F-FMAU(proliferation);
   - SPECT: e.g. (not limited to these) contrast-enhancing units based on radionucleotides such as e.g. ^{99m}Tc, ^{123/5/131}I, ⁶⁷Cu, ⁶⁷Ga, ¹¹¹In, ²⁰¹T1;
   - Therapeutic material: e.g. (not limited to these) toxins, radioisotopes and chemotherapeutics; UV-C emitting nanoparticles such as e.g. YPO₄:Pr; photodynamic therapy (PDT) agents like e.g. compounds based on expanded porphyrin structures; nucleotides for radiotherapy such as e.g. ¹⁵⁷Sm, ¹⁷⁷Lu ^{212/3}Bi, ^{186/8}Re, ⁶⁷Cu, ⁹⁰Y, ¹³¹I' ^{114m}In, At, Ra, Ho;
   - Smart contrast-enhancing units such as e.g. (not limited to these) chemical exchange saturation transfer (CEST); thermosensitive MRI contrast agents (e.g. liposomal); pH sensitive MRI contrast agents; oxygen pressure or enzyme responsive MRI contrast agents; metal ion concentration dependent MRI contrast agents
   - Multi-modality: combinations of the above
- Shell(s) (2): e.g. (not limited to these) may comprise carboxylic acids, acid halides, amines, acid anhydrides, activated esters, maleimides, isothiocyanates, amines, gold, SiO₂ a polyphosphate (e.g. calcium polyphosphate), an amino acid (e.g. cysteine), an organic polymer (e.g. polyethylenglycol/ PEG, polyvinylalcohol/PVA, polyamide, polyacrylat, polyurea), an organic functional polymer (e.g. 1,2-Distearoyl-sn-Glycero-3-Phosphoethanolamine-N-[Carboxy(polyethylene glycol)2000] ammonium salt), a biopolymer (e.g. polysaccharide such as dextran, xylan, glycogen, pectin, cellulose or polypeptide such as collagen, globulin), cysteine or a peptide with high cysteine content or a phospholipid.
- Shell(s) (3): e.g. (not limited to these) comprising or having attached, a polypeptide chain consisting of 1 to 3 cysteines and 4 to 12 basic amino acids selected from the group consisting of arginine, lysine and ornithine or selected from the group consisting of glutamate and aspartate. In addition, further biomolecules like proteins can be incorporated that enable the passage of the complete ensemble through e.g. cell membranes (e.g. the HIV-tat peptide, etc) or increase the biocompatibility or decrease the toxicity. The shell(s) need not be there if the said polypeptide is attached to the contrast-enhancing unit (e.g. the core made of a contrast-enhancing material)
   The core can be separated from the outer shell (3) by 1 to 100 inner shell(s) (2). In preferred embodiments of the present invention, the core can be separated from the outer shell by 1 to 50 inner shell(s). Each of these shells (which can consist of a monolayer or a polylayer of an appropriated material in preferred embodiments of the present invention) has a thickness of about 0.5nm to 100nm. In a preferred embodiment of the present invention, each shell has a thickness of about 0.5nm to 500nm and can be made of different materials or of the same material. Furthermore, the shell can cover the core at least partially.
- Linking unit (4):
   - e.g. (not limited to this) Chimeric polypeptide unit comprising a first and a second polypeptide chain being chemically linked via 1 to 3 cysteine-based disulfide bridges
   - e.g. (not limited to this) The first polypeptide chain consists of 1 to 3 cysteines and 4 to 12 basic amino acids selected from the group consisting of arginine, lysine and ornithine.
   - e.g. (not limited to this) The second polypeptide chain consists of 1 to 3 cysteines and 4 to 12 acidic amino acids selected from the group consisting of glutamate and aspartate.
   - e.g. (not limited to this) One polypeptide chain is linked at its C- or N-terminus to the contrast-enhancing unit or to the outer shell, and the other polypeptide chain is linked to the ligand.
   - e.g. (not limited to this) The chimeric polypeptide unit is chemically linked by 1 to 3 cysteine-based disulfide bridges.
- Ligand (5):
   - e.g. (not limited to this) A ligand, which induces through its specific recognition mechanism the enrichment of contrast agent in distinct tissue or target regions of interest (e.g. by antibody antigen interaction)
   - e.g. (not limited to this) This ligand has attached a polypeptide chain consisting of 1 to 3 cysteines and 4 to 12 basic amino acids selected from the group consisting of arginine, lysine and ornithine or 4 to 12 acidic amino acids selected from the group consisting of glutamate and aspartate.
   - Ligands may be e.g. but are not limited to:

   - Antibodies (monoclonal, polycloncal, mouse, mouse-human chimeric, human, single-chain, diabodies, etc), such as Trastuzumab (breast cancer), Rituximab (non-Hodgkin-lymphoma), Alemtuzumab (chronial-lymphozytic leukemia); Gemtuzumab (acute myelogene leukemia); Edrecolomab (colon cancer); Ibritumomab (non-Hodgkin-lymphoma); Cetuximab (colon cancer); Tositumomab (non-Hodgkin-lymphoma); Epratuzumab (non-Hodgkin-lymphoma); Bevacizumab (lung and colon cancer); anti-CD33 (acute myelogene leukemia); Pemtumomab (ovarian and stomach cancer); Mittumomab (lung and skin cancer); anti-MUC 1 (adenocarcinoma); anti-CEA (adenocarcinoma); anti-CD 64 (plaques), etc
   - Peptides, Polypeptides, Peptidomimetics, such as Somatostatin analogs, vasoactive peptide analogs, neuropeptide Y, RGD peptides, etc
   - Proteins, such as Annexin V, tissue plasminogen activator protein, transporter proteins, etc
   - Macromolecules, e.g., Hyaluronan, Apcitide, Dermatan sulfate
   - Nucleic acids, such as Apatamers, anti-sense DNA/RNA,/PNA, smallinterfering RNAs, etc
   - Lipids, such as Phospholipids, etc
   - Lectins, e.g. Leukocyte stimulatiry lectin
   - Saccharides

### Figure 2:

Reaction scheme for the surface modification of a contrast-enhancing unit with a Cys-Glu-Glu-Glu-Glu-Glu-Glu-Glu-Glu (= Cys-Glu8) functionality by a one pot reaction of carboxylic acids, linked to the contrast-enhancing unit, with 1-Ethyl-3-(dimethylaminopropyl) carbodiimide hydrochloride (EDC) to form a o-Acylisourea intermediate (room temperature, pH ≈ 5). This intermediate reacts with sulfo-NHS to give a sulfo-NHS-ester intermediate. The excess of EDC is quenched by the addition of 2-mercaptoethanol. Finally the reaction with a primary amine containing Cys-Glu8 leads to the desired amide bond (room temperature, pH ≈ 7).
- Core (1) = contrast-enhancing unit: CdSe/ZnS Quantum Dots
- Shell(s) (2): carboxylic acids
- Shell(s) (3): an amine terminated Cys-Glu8 polypeptide chain

### Figure 3:

Reaction scheme for the coupling of Cys-Lys-Lys-Lys-Lys-Lys-Lys-Lys-Lys (=Cys-Lys8) to contrast-enhancing material bearing a Cys-Glu8 functionality by a one pot reaction of Cys-Lys8 and a contrast-enhancing material bearing a Cys-Glu8 functionality, followed by the disulfide bond formation, due to the addition of an oxidation agent, GSSG (oxidized Glutathione).
- Core (1) = contrast-enhancing unit: CdSe/ZnS Quantum Dots
- Shell(s) (2): carboxylic acids
- Shell(s) (3): an amine terminated Cys-Glu8 polypeptide chain

### Example:

CdSe/ZnS Quantum Dots (contrast-enhancing units) are surface modified with a carboxylic acid functionality by an acid by a water soluble polymer bearing a carboxylic acid function at one end and a 1.2-Distearoyl-sn-Glycero-3-Phosphoethanolamine function at the other end.

The COOH coated Quantum Dots are obtained by mixing (4h at 50 °C):
- 100µl CdSe/ZnS (in Chloroform, 1 w/v %)
- 100µl Chloroform
- 200µl DPPC (5 mM) - DPPC = 1.2-Dipalmitoyl-sn-Gycero-3-phosphocholine
- 200µl DSPE-PEG2000-COOH (5 mM) - DSPE-PEG2000-COOH: 1.2-Distearoyl-sn-Glycero-3-Phosphoethanolamine-N-[Carboxy(polyethylene glycol)2000] ammonium salt

And finally removing the Chloroform by vacuum and dispersing the COOH coated Quantum Dots in water by an ultrasonic treatment.

The 1.2-Distearoyl-sn-Glycero-3-Phosphoethanolamine-N-[Carboxy(polyethylene glycol)2000] ammonium salt binds to the surface of the nanoparticles by hydrophobic interactions ( or adsorption)by the 1.2-Distearoyl-sn-Glycero-3-Phosphoethanolamine end group. Furthermore the 1.2-Distearoyl-sn-Glycero-3-Phosphoethanolamine-N-[Carboxy(polyethylene glycol)2000] ammonium salt provides a carboxy function, which is protonated, at an acid pH, to obtain a carboxylic acid.

DPPC is used as a dummy (or spacer) to leave spaces between the COOH functions fixed on the nanoparticles. Actually the covering of the whole nanoparticle surface only by COOH functions could have adverse effects by creating interactions, and therefore contrast, in undesired tissues or undesired areas of the body.

The contrast-enhancing unit is surface modified with Cys-Glu8 functionality by a coupling via an acid.

Other examples would be e.g. coupling via an activated ester, via maleimide or via isothiocyanate.

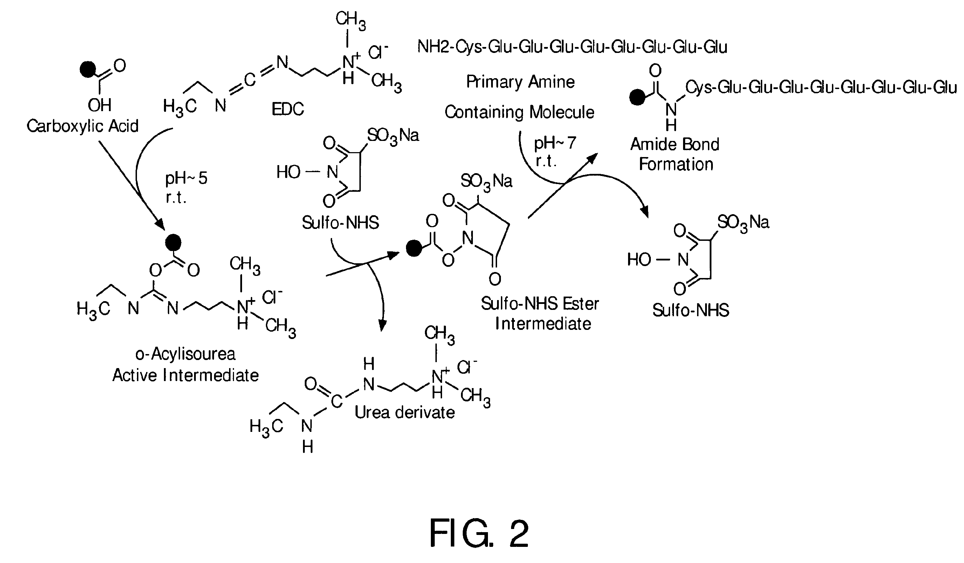

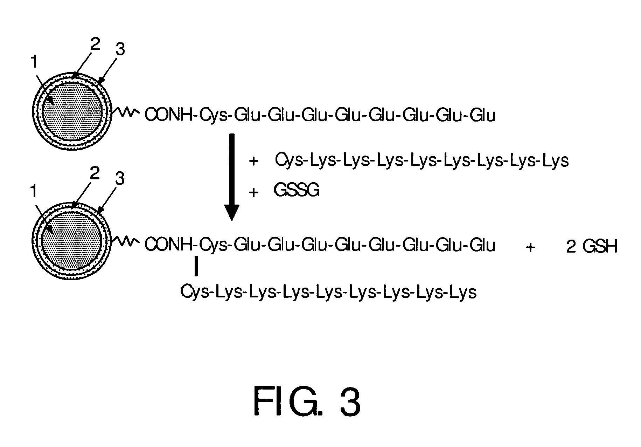

This can be done by:
1) Modifying water soluble CdSe/ZnS Quantum Dots (Fig. 2):
   - 55µl water
   - 40µl 10x PBS solution (PBS = phosphat buffer saline: 0.01 M phosphate buffer, 0.0027 M potassium chloride, 0.137 M sodium chloride, pH 7.4)
   - 100µl0.1 M EDC solution (EDC = 1-Ethyl-3-(dimethylaminopropyl) carbodiimide hydrochloride)
   - 5µl 20mM sulfo-NHS solution (N-Hydroxysulfosuccinimide sodium salt)
   - 200µl 2µM CdSe/ZnS Quantum Dots(COOH terminated) solution
   - Incubation at room temperature (30 min)
   - 10µl 2-mercaptoethanol
   - mixing for 15mins
   - 50µl 20mM Cys-Glu8
   - mixing at r.t. (2h)
   - separation of QDs by centrifugation
2) Coupling of Cys-Lys8 to contrast-enhancing unit QD-Cys-Glu8 unit (Fig. 3): aqueous solution comprising:
   - 50µM Cys-Lys8
   - 5µM QD-Cys-Glu8
   - 2mM EDTA
   - 2.5mM oxidized Glutathione (GSSG)
   - Incubation at room temperature (5h)
   - separation of QD-conjugates by ultracentrifugation

## Claims

1. A method for the production of a targeting contrast agent or targeting therapeutic agent, the method comprising the steps of:
a) providing a core;
b) adding a shell to the core;
c) modifying the shell through attaching at least a first polypeptide comprising at least one cysteine;
d) providing a ligand bearing a second complementary polypeptide comprising at least one cysteine; and
e) linking at least one ligand to the shell via a linking unit which is formed through electrostatic association between said first polypeptide and said second complementary polypeptide followed by at least one disulfide bond formation between said cysteines.

2. The method according to claim 1, wherein in step b) more than one shell is added to the core.

3. The method according to any one of claims 1 or 2, wherein the shell/shells comprises/comprise a monolayer or a polylayer.

4. The method according to any one of claims 1 to 3, wherein each shell comprises the same material or different material.

5. The method according to any one of claims 1 to 4, wherein the shell/shells covers/cover the core at least partially.

6. A method for the production of a targeting contrast agent comprising the steps of:
a) providing a core;
b) modifying the core through attaching at least a first polypeptide comprising at least one cysteine;
c) providing a ligand bearing a second complementary polypeptide comprising at least one cysteine; and
d) linking at least one ligand to the core via a linking unit which is formed through electrostatic association between said first polypeptide and said second complementary polypeptide followed by a disulfide bond formation between said cysteines.

7. The method according to any one of the claims 1 to 6 wherein:
a) the first polypeptide comprises 1 to 3 cysteines and 4 to 12 basic amino acids selected from the group consisting of arginine, lysine and ornithine or 4 to 12 acidic amino acids selected from the group consisting of glutamate and aspartate; and
b) the second complementary polypeptide comprises of 1 to 3 cysteines and 4 to 12 acidic amino acids selected from the group consisting of glutamate and aspartate or 4 to 12 basic amino acids selected from the group consisting of arginine, lysine and ornithine, wherein the group of basic amino acids selected for the first and second polypeptide are different.

8. A method according to any one of the claims 1 to 7 wherein the first polypeptide is linked at its C- or N-terminus to the shell or core and the second complementary polypeptide is linked at its C- or N-terminus to the ligand.

9. A method according any one of the claims 1 to 8 wherein the material used as the core is selected from:
ferro-, antiferro-, ferrimagnetic or superparamagnetic material such as iron (Fe), iron oxide γ-Fe2O3 or Fe₃O₄ or ferrit with spinell structure MFe₂O₄ (M = Mn, Co, Ni, Cu, Zn, Cd) or ferrit with granat structure M₃FeₛO₁₂ (M = Y, La, Ce, Pr, Nd, Sm, Eu, Gd, Tb, Dy, Ho, Er, Tm, Yb, Lu) or ferrit with a magnetoplumbit structure MFe₁₂O₁₉ (M = Ca, Sr, Ba, Zn) or other hexagonal ferrit structures such as e.g. Ba₂M₂Fe₁₂O₂₂ (M = Mn, Fe, Co, Ni, Zn, Mg); in all cases the core can be doped with additional 0.01 to 5.00mol-% of Mn, Co, Ni, Cu, Znor F; Paramagnetic ion (e.g. lanthanide, manganese, iron, copper) based contrast-enhancing units e.g. Gadolinium chelates such as Gd(DTPA), Gd(BMA-DTPA), Gd(DOTA), Gd(DO3A); oligomeric structures; macromolecular structures such as Albumin Gd(DTPA)20-35, Dextran Gd(DTPA), Gd(DTPA)-24-cascade polymer, polylysine-Gd(DTPA), MPEG polylysine-Gd(DTPA); dendrimeric structures of lanthanide-based contrast-enhancing units; Manganese-based contrast-enhancing units such as Mn(DPDP), Mn(EDTA-MEA), poly-Mn(EED-EEA), and polymeric structures; liposomes as carriers of paramagnetic ions e.g. liposomal Gd(DTPA); non-proton imaging agents.

10. A method according any one of the claims 1 to 5 and 7 to 8 wherein the material used as the core is selected from:
luminescent material such as nanophosphores (e.g. rare earth doped YPO₄ or LaPO₄) or semiconducting nanocrystals (so called quantum dots; e.g. CdS, CdSe, ZnS/CdSe, ZnS/CdS); carbocyanine dyes; tetrapyrrole-based dyes (porphyrins, chlorins, phthalocyanines and related structures); deltaaminolevulinic acid; fluorescent lanthanide chelates; fluorescein or 5- aminofluorescein or fluorescein-isothiocyanate (FITC) or other fluorescein-related fluorophors such as Oregon Green, naphthofluorescein.

11. A method according to any one of the claims 1 to 8 wherein the material used as the core is selected from:
encapsulated gas (e.g. air, perfluorpropane, dodecafluorcarbon, sulphur hexafluride, perfluorcarbon) bubbles (such as Optison from Amersham, Levovist from Schering); encapsulated droplets; nanoparticles (e.g. platinum, gold, tantalum).

12. A method according to any one of the claims 1 to 5 and 7 to 8 wherein the material used as the core is selected from:
iodinated contrast-enhancing units such as e.g. ionic and non-ionic derivatives of 2,4,6-tri-iodobenzene; barium sulfate-based contrast-enhancing units; metal ion chelates such as e.g. gadolinium-based compounds; boron clusters with high proportion of iodine; polymers such as iodinated polysaccharides, polymeric triiodobenzenes; particles from iodinated compounds displaying low water solubility; liposomes containing iodinated compounds; iodinated lipids such as triglycerides, fatty acids.

13. A method according to any one of the claims 1 to 8 wherein the material used as the core is selected from:
11^{C}, ¹³N, ¹⁵O, ^{66/8}Ga, ⁶⁰CU, ⁵²Fe, ⁵⁵Co, ^{61/2/4}Cu, ^{62/3}Zn, ^{70/1/4}AS, ^{75/6}Br, ⁸²Rb, ⁸⁶Y, ⁸⁹Zr, ¹¹⁰In, ^{120/4}I, ¹²²Xe and ¹⁸F based tracers, such as e.g. ¹⁸F-FDG (glucose metabolism); ¹¹C-Methionine, ¹¹C-Tyrosine, ¹⁸F-FMT, ¹⁸F-FMTor ¹⁸F-FET (amino acids); ¹⁸F-FMISO, ⁶⁴Cu-ATSM (hypoxia); ¹⁸F-FLT, ¹¹C-Thymidine, ¹⁸F-FMAU(proliferation).

14. A method according to any one of the claims 1 to 8 wherein the material used as the core is selected from:
contrast-enhancing units based on radionucleotides such as e.g. ^{99m}Tc, ^{123/5/131}I, ⁶⁷Cu, ⁶⁷Ca, ¹¹¹In, ²⁰¹T1.

15. A method according to any one of the claims 1 to 5 and 7 to 8 wherein the material used as the core is selected from:
toxins, radioisotopes and chemotherapeutics; UV-C emitting nanoparticles such as e.g. YPO₄:Pr; photodynamic therapy (PDT) agents such as e.g. compounds based on expanded porphyrin structures; nucleotides for radiotherapy such as e.g. ¹⁵⁷Sm, ¹⁷⁷Lu, ^{212/3}Bi, ^{186/8}Re, ⁶⁷Cu, ^{9O}Y, ¹³¹I'^{114m}In, At, Ra, Ho.

16. A method according to any one of the claims 1 to 8 wherein the material used as the core is selected from:
chemical exchange saturation transfer (CEST); thermosensitive MRI contrast agents (e.g. liposomal); pH sensitive MRI contrast agents; oxygen pressure or enzyme responsive MRI contrast agents; metal ion concentration dependent MRI contrast agents.

17. A method according to any any of the claims 1 to 16 wherein the material used as the core is a combination of two or more materials.

18. A method according to any any of the claims 1 to 5 and 7 to 17 wherein the material used as shell(s) is selected from:
carboxylic acids, acid halides, amines, acid anhydrides , activated esters, maleimides, isothiocyanates, amines, gold, SiO₂, lipids, surfactants, a polyphosphate (e.g. calcium polyphosphate), an amino acid (e.g. cysteine), an organic polymer (e.g. polyethylenglycol/ PEG, polyvinylalcohol/PVA, polyamide, polyacrylat, polyurea), an organic polymer with functional end groups (e.g. 1,2-Distearoyl-sn-Glycero-3-Phosphoethanolamine-N-[Carboxy(polyethylene glycol)2000] ammonium salt), a biopolymer (e.g. polysaccharide such as dextran, xylan, glycogen, pectin, cellulose or polypeptide such as collagen, globulin), cysteine or a peptide with high cysteine content or a phospholipid.

19. A method according to any one of the claims 1 to 5 and 7 to 17 wherein further components can be incorporated into the shell(s).

20. A method according to any one of the claims 1 to 19 wherein the polypeptides of the linking unit are chemically linked via 1 to 3 cysteine-based disulfide bonds.

21. A method according to any one of the claims 1 to 20 wherein the material used as ligand is selected from:
Antibodies (monoclonal, polycloncal, mouse, mouse-human chimeric, human, single-chain, diabodies, etc), such as Trastuzumab (breast cancer), Rituximab (non-Hodgkin-lymphoma), Alemtuzumab (chronial-lymphozytic leukemia); Gemtuzumab (acute myelogene leukemia); Edrecolomab (colon cancer); Ibritumomab (non-Hodgkin-lymphoma); Cetuximab (colon cancer); Tositumomab (non-Hodgkin-lymphoma); Epratuzumab (non-Hodgkin-lymphoma); Bevacizumab (lung and colon cancer); anti-CD33 (acute myelogene leukemia); Pemtumomab (ovarian and stomach cancer); Mittumomab (lung and skin cancer); anti-MUC 1 (adenocarcinoma); anti-CEA (adenocarcinoma); anti-CD 64 (plaques; Peptides, Polypeptides, Peptidomimetics, such as Somatostatin analogs, vasoactive peptide analogs, neuropeptide Y, RGD peptides; Proteins, such as Annexin V, tissue plasminogen activator protein, transporter proteins; Macromolecules, e.g., Hyaluronan, Apcitide, Dermatan sulphate; Nucleic acids, such as Apatamers, anti-sense DNA/RNA,/PNA, smallinterfering RNAs; Lipids, such as Phospholipids; Lectins, e.g. Leukocyte stimulatiry lectin and Saccharides.

22. Targeting contrast agents or targeting therapeutic agents produced by a method according to any of claims 1 to 21.

23. Targeting contrast agents or targeting therapeutic agents according to any one of claim 22 for use in diagnosis or therapy.

24. Targeting contrast agents or targeting therapeutic agents according to any one of claims 22 to 23 for use in targeting molecular imaging.

25. Targeting contrast agents according to any one of claims 22 to 23 for use in CT, MRI, PET, SPECT or US.

26. Use of the targeting contrast agents or targeting therapeutic agents according to any one of claims 22 to 23 for the production of compounds suitable in diagnosis or therapy.

27. Use of the targeting contrast agents or targeting therapeutic agents according to any one of claims 22 to 23 for the production of compounds suitable for targeting molecular imaging.

28. Use of the targeting contrast agents according to any one of claims 22 to 23 for the production of compounds suitable in CT, MRI, PET, SPECT or US.

## Patentansprüche

1. Verfahren zur Herstellung eines Kontrast-Targetingmittels oder therapeutischen Targetingmittels, wobei das Verfahren die folgenden Schritte umfasst:
a) Vorsehen eines Kerns;
b) Zugeben einer Hülle zu dem Kern;
c) Modifizieren der Hülle durch Anlagerung von zumindest einem ersten Polypeptid mit mindestens einem Cystein;
d) Vorsehen eines Liganden, der ein zweites, komplementäres Polypeptid mit mindestens einem Cystein trägt; sowie
e) Verbinden von mindestens einem Liganden mit der Hülle mit Hilfe einer Verbindungseinheit, die, gefolgt von mindestens einer Disulfidverbrückung zwischen den Cysteinen, durch elektrostatische Assoziation zwischen dem ersten Polypeptid und dem zweiten, komplementären Polypeptid gebildet wird.

2. Verfahren nach Anspruch 1, wobei in Schritt b) mehr als eine Hülle zu dem Kern zugegeben wird.

3. Verfahren nach Anspruch 1 oder 2, wobei die Hülle/Hüllen eine Einfachschicht oder eine Mehrfachschicht umfasst/umfassen.

4. Verfahren nach einem der Ansprüche 1 bis 3, wobei jede Hülle das gleiche Material oder unterschiedliches Material umfasst.

5. Verfahren nach einem der Ansprüche 1 bis 4, wobei die Hülle/Hüllen den Kern zumindest teilweise bedeckt/bedecken.

6. Verfahren zur Herstellung eines Kontrast-Targetingmittels, welches die folgenden Schritte umfasst:
a) Vorsehen eines Kerns;
b) Modifizieren des Kerns durch Anlagerung von zumindest einem ersten Polypeptid mit mindestens einem Cystein;
c) Vorsehen eines Liganden, der ein zweites, komplementäres Polypeptid mit mindestens einem Cystein trägt; sowie
d) Verbinden von mindestens einem Liganden mit dem Kern mit Hilfe einer Verbindungseinheit, die, gefolgt von einer Disulfidverbrückung zwischen den Cysteinen, durch elektrostatische Assoziation zwischen dem ersten Polypeptid und dem zweiten, komplementären Polypeptid gebildet wird.

7. Verfahren nach einem der Ansprüche 1 bis 6, wobei:
a) das erste Polypeptid 1 bis 3 Cysteine und 4 bis 12 basische Aminosäuren, die aus der Gruppe, bestehend aus Arginin, Lysin und Ornithin, ausgewählt werden, oder 4 bis 12 saure Aminosäuren, die aus der Gruppe, bestehend aus Glutamat und Aspartat, ausgewählt werden, umfasst; und
b) das zweite, komplementäre Polypeptid 1 bis 3 Cysteine und 4 bis 12 saure Aminosäuren, die aus der Gruppe, bestehend aus Glutamat und Aspartat, ausgewählt werden, oder 4 bis 12 basische Aminosäuren, die aus der Gruppe, bestehend aus Arginin, Lysin und Ornithin, ausgewählt werden, umfasst, wobei die Gruppe von basischen Aminosäuren, die für das erste und zweite Polypeptid ausgewählt werden, unterschiedlich sind.

8. Verfahren nach einem der Ansprüche 1 bis 7, wobei das erste Polypeptid an seinem C- oder N-Terminus mit der Hülle oder dem Kern verbunden ist und das zweite, komplementäre Polypeptid an seinem C- oder N-Terminus mit dem Liganden verbunden ist.

9. Verfahren nach einem der Ansprüche 1 bis 8, wobei das als Kern verwendete Material ausgewählt wird aus:
ferro-, antiferro-, ferrimagnetischem oder superparamagnetischem Material, wie z.B. Eisen (Fe), Eisenoxid y-Fe203 oder Fe₃O₄ oder Ferrit mit Spinellstruktur MFe₂O₄ (M = Mn, Co, Ni, Cu, Zn, Cd) oder Ferrit mit Granatstruktur M₃Fe₅O₁₂ (M = Y, La, Ce, Pr, Nd, Sm, Eu, Gd, Tb, Dy, Ho, Er, Tm, Yb, Lu) oder Ferrit mit einer Magnetoplumbitstruktur MFe₁₂O₁₉ (M = Ca, Sr, Ba, Zn) oder anderen hexagonalen Ferritstrukturen, wie z.B. Ba₂M₂Fe₁₂O₂₂ (M = Mn, Fe, Co, Ni, Zn, Mg); in sämtlichen Fällen kann der Kern mit zusätzlichen 0,01 bis 5,00 Mol-% Mn, Co, Ni, Cu, Znor F dotiert sein; Kontrastverbesserungseinheiten auf der Basis paramagnetischer Ionen (z.B. Lanthanoid, Mangan, Eisen, Kupfer), z.B. Gadoliniumchelat, wie z.B. Gd(DTPA), Gd(BMA-DTPA), Gd(DOTA), Gd(DO3A); oligomere Strukturen; makromolekulare Strukturen, wie z.B. Albumin Gd(DTPA)20-35, Dextran Gd(DTPA), Gd(DTPA)-24-Kaskaden-Polymer, Polylysin-Gd(DTPA), MPEG-Polylysin-Gd(DTPA); dendrimerische Strukturen von Lanthanoid-basierten Kontrastverbesserungseinheiten; Mangan-basierte Kontrastverbesserungseinheiten, wie z.B. Mn(DPDP), Mn(EDTA-MEA), Poly-Mn(EED-EEA) sowie polymere Strukturen; Liposomen als Träger von paramagnetischen Ionen, z.B. liposomales Gd(DTPA); Nicht-Proton-Bilderzeugungsmittel.

10. Verfahren nach einem der Ansprüche 1 bis 5 und 7 bis 8, wobei das als Kern verwendete Material ausgewählt wird aus:
lumineszierendem Material, wie z.B. Nanophosphore (z.B. mit Seltenen Erden dotiertes YPO₄ oder LaPO₄) oder halbleitende Nanokristalle (so genannte Quantenpunkte; z.B. CdS, CdSe, ZnS/CdSe, ZnS/CdS); Carbocyanin-Farbstoffe; Tetrapyrrol-basierte Farbstoffe (Porphyrine, Chlorine, Phthalocyanine und verwandte Strukturen); Deltaaminolävulinsäure; Fluoreszenz-Lanthanoidchelate; Fluorescein oder 5-Aminofluorescein oder Fluorescein-Isothiocyanat (FITC) oder andere Fluoresceinverwandte Fluorophore, wie z.B. Oregon Green, Naphthofluorescein.

11. Verfahren nach einem der Ansprüche 1 bis 8, wobei das als Kern verwendete Material ausgewählt wird aus:
gekapselten Gas- (z.B. Luft, Perfluorpropan, Dodecafluorkohlenstoff, Schwefelhexafluorid" Perfluorkohlenstoff) Blasen (wie z.B. Optison von Amersham, Levovist von Schering); gekapselten, kleinen Tropfen; Nanopartikeln (z.B. Platin, Gold, Tantal).

12. Verfahren nach einem der Ansprüche 1 bis 5 und 7 bis 8, wobei das als Kern verwendete Material ausgewählt wird aus:
iodierten Kontrastverbesserungseinheiten, wie z.B. ionischen und nicht ionischen Derivaten des 2,4,6-tri-iodbenzols; Bariumsulfat-basierten Kontrastverbesserungseinheiten; Metallionenchelaten, wie z.B. Gadolinium-basierten Verbindungen; Borclustern mit hohem Iodanteil; Polymeren, wie z.B. iodierten Polysacchariden, polymeren Triiodbenzolen; Teilchen von iodierten Verbindungen, die eine geringe Wasserlöslichkeit aufweisen; Liposomen enthaltenden, iodierten Verbindungen; iodierten Lipiden, wie z.B. Triglyceriden, Fettsäuren.

13. Verfahren nach einem der Ansprüche 1 bis 8, wobei das als Kern verwendete Material ausgewählt wird aus:
¹¹C, ¹³N, ¹⁵O, ^{66/8}Ga, ⁶⁰Cu, ⁵²Fe, ⁵⁵Co, ^{61/2/4}Cu, ^{62/3}Zn, ^{70/1/4}As, ^{75/6}Br, ⁸²Rb, ⁸⁶Y, ⁸⁹Zr, ¹¹⁰In, ^{120/4}I_{,} ¹²²Xe und ¹⁸F-basierten Tracern, wie z.B. ¹⁸F-FDG (Glucosestoffwechsel); ¹¹C-Methionin, ¹¹C-Tyrosin, ¹⁸F-FMT, ¹⁸F-FMT oder ¹⁸F-FET (Aminosäuren); ¹⁸F-FMISO , ⁶⁴Cu-ATSM (Hypoxie), ¹⁸F-FLT, ¹¹C-Thymidin, ¹⁸F-FMAU (Proliferation).

14. Verfahren nach einem der Ansprüche 1 bis 8, wobei das als Kern verwendete Material ausgewählt wird aus:
Kontrastverbesserungseinheiten, die auf Radionucleotiden, wie z.B. ^{99m}Tc, ^{123/5/131}I, ⁶⁷Cu, ⁶⁷Ga, ¹¹¹In, ²⁰¹T1, basieren.

15. Verfahren nach einem der Ansprüche 1 bis 5 und 7 bis 8, wobei das als Kern verwendete Material ausgewählt wird aus:
Toxinen, Radioisotopen und Chemotherapeutik; UV-C-emittierenden Nanopartikeln, wie z.B. YPO₄:Pr; photodynamischen Therapie-(PDT) Mitteln, wie z.B. Verbindungen, die auf erweiterten Porphyrinstrukturen basieren; Nucleotiden zur Radiotherapie, wie z.B. ¹⁵⁷Sm, ¹⁷⁷Lu, ^{212/3}Bi, ^{186/8}Re, ⁶⁷Cu, ⁹⁰Y, ¹³¹I, ^{114m}In, At, Ra, Ho.

16. Verfahren nach einem der Ansprüche 1 bis 8, wobei das als Kern verwendete Material ausgewählt wird aus:
,Chemical Exchange Saturation Transfer" (CEST); wärmeempfindlichen Kontrastmitteln (z.B. liposomal); pH-empfindlichen MRI-Kontrastmitteln; Sauerstoffdruck- oder Enzym-responsiven MRI-Kontrastmitteln; MRI-Kontrastmitteln, die von der Metallionenkonzentration abhängig sind.

17. Verfahren nach einem der Ansprüche 1 bis 16, wobei das als Kern verwendete Material eine Kombination aus zwei Materialien oder mehr ist.

18. Verfahren nach einem der Ansprüche 1 bis 5 und 7 bis 17, wobei das als Hülle(n) verwendete Material ausgewählt wird aus:
Carbonsäuren, Säurehalogeniden, Aminen, Säureanhydriden, aktivierten Estern, Maleimiden, Isothiocyanaten, Aminen, Gold, SiO₂, Lipiden, oberflächenaktiven Stoffen, einem Polyphosphat (z.B. Calciumpolyphosphat), einer Aminosäure (z.B. Cystein), einem organischen Polymer (z.B. Polyethylenglycol/PEG, Polyvinylalkohol/PVA, Polyamid, Polyacrylat, Polyharnstoff), einem organischen Polymer mit funktionellen Endgruppen (z.B. 1,2-Distearoyl-sn-Glycero-3-Phosphoethanolamin-N-[Carboxy(polyethylenglycol)2000] Ammoniumsalz), einem Biopolymer (z.B. Polysaccharid, wie z.B. Dextran, Xylan, Glycogen, Pektin, Cellulose oder Polypeptid, wie z.B. Collagen, Globulin), Cystein oder einem Peptid mit hohem Cysteingehalt oder einem Phospholipid.

19. Verfahren nach einem der Ansprüche 1 bis 5 und 7 bis 17, wobei weitere Komponenten in die Hülle(n) inkorporiert werden können.

20. Verfahren nach einem der Ansprüche 1 bis 19, wobei die Polypeptide der Verbindungseinheit über 1 bis 3 Cystein-basierte Disulfidverbindungen chemisch verbunden sind.

21. Verfahren nach einem der Ansprüche 1 bis 20, wobei das als Ligand verwendete Material ausgewählt wird aus:
Antikörpern (monoklonal, polyklonal, Maus, Maus-Human, chimer, Human, einzelkettig, Diabodies usw.), wie z.B. Trastuzumab (Brustkrebs), Rituximab (nicht lymphogranulomatöses Lymphom), Alemtuzumab (chronische lymphatische Leukämie); Gemtuzumab (akute myelogene Leukämie); Edrecolomab (Darmkrebs); Ibritumomab (nicht lymphogranulomatöses Lymphom); Cetuximab (Darmkrebs); Tositumomab (nicht lymphogranulomatöses Lymphom); Bevacizumab (Lungen- und Darmkrebs); anti-CD33 (akute myelogene Leukämie); Pemtumomab (Eierstock- und Magenkrebs); Mittumomab (Lungen- und Hautkrebs); anti-MUC 1 (Drüsenkrebs); anti-CEA (Drüsenkrebs); anti-CD 64 (Plaques; Peptide, Polypeptide, Peptidomimetika, wie z.B. Somatostatin-Analoga, vasoaktive Peptidanaloga, Neuropeptid Y, RGD-Peptide; Proteinen, wie z.B. Annexin V, Tissue-Plasminogen-Activator-Protein, Transporter-Proteine; Makromolekülen, z.B. Hyaluronan, Apcitide, Dermatansulfat; Nukleinsäuren, wie z.B. Aptamere, Antisense DNA/RNA/PNA, Smallinterfering RNAs; Lipiden, wie z.B. Phospholipide; Lektine, z.B. leukozytenstimulierendes Lektin und Saccharide.

22. Kontrast-Targetingmittel oder therapeutische Targetingmittel, die durch ein Verfahren nach einem der Ansprüche 1 bis 21 hergestellt werden.

23. Kontrast-Targetingmittel oder therapeutische Targetingmittel nach Anspruch 22 zur Verwendung bei Diagnose oder Therapie.

24. Kontrast-Targetingmittel oder therapeutische Targetingmittel nach einem der Ansprüche 22 bis 23 zur Verwendung beim Targeting molekularer Bilderzeugung.

25. Kontrast-Targetingmittel nach einem der Ansprüche 22 bis 23 zur Verwendung bei CT, MRI, PET, SPECT oder US.

26. Einsatz von Kontrast-Targetingmitteln oder therapeutischen Targetingmitteln nach einem der Ansprüche 22 bis 23 zur Herstellung von Verbindungen, die bei Diagnose oder Therapie geeignet sind.

27. Einsatz von Kontrast-Targetingmitteln oder therapeutischen Targetingmitteln nach einem der Ansprüche 22 bis 23 zur Herstellung von Verbindungen, die beim Targeting molekularer Bilderzeugung geeignet sind.

28. Einsatz von Kontrast-Targetingmitteln nach einem der Ansprüche 22 bis 23 zur Herstellung von Verbindungen, die bei CT, MRI, PET, SPECT oder US geeignet sind.

## Revendications

1. Procédé de production d'un agent de contraste de ciblage ou d'un agent thérapeutique de ciblage, le procédé comprenant les étapes suivantes consistant à :
a) fournir un noyau ;
b) ajouter une coquille au noyau ;
c) modifier la coquille par l'association d'au moins un premier polypeptide comprenant au moins une cystéine ;
d) fournir un ligand qui contient un deuxième polypeptide complémentaire comprenant au moins une cystéine ; et
e) lier au moins un ligand à la coquille par le biais d'une unité de liaison qui est formée par l'association électrostatique entre ledit premier polypeptide et ledit deuxième polypeptide complémentaire, ce qui est suivi d'au moins une formation de liants de disulfure entre lesdites cystéines.

2. Procédé selon la revendication 1, dans lequel, dans l'étape b), plus d'une coquille est ajoutée au noyau.

3. Procédé selon l'une quelconque des revendications précédentes 1 ou 2, dans lequel la (les) coquille(s) comprend (comprennent) une monocouche ou une poly-couche.

4. Procédé selon l'une quelconque des revendications précédentes 1 à 3, dans lequel chaque coquille comprend le même matériau ou du matériau différent.

5. Procédé selon l'une quelconque des revendications précédentes 1 à 4, dans lequel la (les) coquille(s) couvre(nt) au moins partiellement le noyau.

6. Procédé de production d'un agent de contraste de ciblage comprenant les étapes suivantes consistant à :
a) fournir un noyau ;
b) modifier le noyau par l'association d'au moins un premier polypeptide comprenant au moins une cystéine ;
c) fournir un ligand qui contient un deuxième polypeptide complémentaire comprenant au moins une cystéine ; et
e) lier au moins un ligand au noyau par le biais d'une unité de liaison qui est formée par l'association électrostatique entre ledit premier polypeptide et ledit deuxième polypeptide complémentaire, ce qui est suivi d'au moins une formation de liants de disulfure entre lesdites cystéines.

7. Procédé selon l'une quelconque des revendications précédentes 1 à 6, dans lequel :
a) le premier polypeptide comprend 1 à 3 cystéines et 4 à 12 acides aminés de base qui sont sélectionnés parmi le groupe constitué d'arginine, de lysine et d'ornithine ou 4 à 12 acides aminés acides qui sont sélectionnés parmi le groupe constitué de glutamate et d'aspartate ; et
b) le deuxième polypeptide complémentaire comprend 1 à 3 cystéines et 4 à 12 acides aminés acides qui sont sélectionnés parmi le groupe constitué de glutamate et d'aspartate ou 4 à 12 acides aminés de base qui sont sélectionnés parmi le groupe constitué d'arginine, de lysine et d'omithine, dans lequel le groupe d'acides aminés de base qui sont sélectionnés pour le premier et le deuxième polypeptide sont différents.

8. Procédé selon l'une quelconque des revendications précédentes 1 à 7, dans lequel le premier polypeptide est lié, à son terminus C ou N, à la coquille ou au noyau et dans lequel le deuxième polypeptide complémentaire est lié, à son terminus C ou N, au ligand.

9. Procédé selon l'une quelconque des revendications précédentes 1 à 8, dans lequel le matériau qui est utilisé en tant que noyau est sélectionné parmi :
le matériau ferromagnétique, antiferromagnétique, ferrimagnétique ou superparamagnétique, tel que du fer (Fe), de l'oxyde de fer γ-Fe₂O₃ ou Fe₃O₄ ou de la ferrite avec une structure spinelle MFe₂O₄ (M = Mn, Co, Ni, Cu, Zn, Cd) ou de la ferrite avec une structure grenat M₃FeₛO₁₂ (M = Y, La, Ce, Pr, Nd, Sm, Eu, Gd, Tb, Dy, Ho, Er, Tm, Yb, Lu) ou de la ferrite avec une structure magnétoplumbite MFe₁₂O₁₉ (M = Ca, Sr, Ba, Zn) ou d'autres structures hexagonales de ferrite, telles que, par exemple, Ba₂M₂Fe₁₂O₂₂ (M = Mn, Fe, Co, Ni, Zn, Mg) ; dans tous les cas le noyau peut être dopé d'une quantité additionnelle de 0,01 à 5,00 % en moles de Mn, de Co, de Ni, de Cu, de Znor F ; des unités d'amélioration de contraste, par exemple, des chélates de gadolinium, tels que Gd(DTPA), Gd(BMA-DTPA), Gd(DOTA), Gd(D03A) à base d'ions paramagnétiques (par exemple, du lanthanide, du manganèse, du fer, du cuivre) ; des structures oligomères, des structures macromoléculaires, telles que l'albumine Gd(DTPA)20-35, le dextran Gd(DTPA), le polymère Gd(DTPA)-24-cascade, la polylysine-Gd(DTPA), la polylysine MPEG-Gd(DTPA) ; des structures dendrimères d'unités d'amélioration de contraste à base de lanthanide ; des unités d'amélioration de contraste à base de manganèse, telles que Mn(DPDP), Mn(EDTA-MEA), poly-Mn(EED-EEA), et des structures polymères ; des liposomes en tant que porteurs d'ions paramagnétiques, par exemple, le Gd(DTPA) liposomal ; des agents non-protons d'imagerie.

10. Procédé selon l'une quelconque des revendications précédentes 1 à 5 et 7 à 8, dans lequel le matériau qui est utilisé en tant que noyau est sélectionné parmi :
du matériau luminescent, tel que des nanophosphores (par exemple, YPO₄ ou LaPO₄ dopé de terre rare) ou des nanocristaux semi-conducteurs (des soi-disant points quantiques ; par exemple, CdS, CdSe, ZnS/CdSe, ZnS/CdS) ; des colorants de carbocyanine ; des colorants à base de tétrapyrrole (des porphyrines, des chlores, des phtalocyanines et des structures apparentées) ; de l'acide delta-aminolévulinique ; des chélates de lanthanide fluorescents ; de la fluorescéine ou de la 5-aminofluorescéine ou de l'isothiocyanate de fluorescéine (FITC) ou d'autres fluorophores liés à fluorescéine, tels que le vert d'Oregon, de la naphtofluorescéine.

11. Procédé selon l'une quelconque des revendications précédentes 1 à 8, dans lequel le matériau qui est utilisé en tant que noyau est sélectionné parmi :
des bulles de gaz encapsulées (par exemple, d'air, de perfluoropropane, de dodécafluorocarbone, d'hexafluride de soufre, de perfluorocarbone), telles qu'Optison d'Amersham, Levovist de Schering) ; des gouttelettes encapsulées ; des nanoparticules (par exemple, du platine, de l'or, du tantale).

12. Procédé selon l'une quelconque des revendications précédentes 1 à 5 et 7 à 8, dans lequel le matériau qui est utilisé en tant que noyau est sélectionné parmi :
des unités iodées d'amélioration de contraste, telles que des dérivés ioniques et non ioniques de 2,4,6-tri-iodobenzène ; des unités d'amélioration de contraste à base de sulfate de baryum ; des chélates d'ions métalliques, tels que, par exemple, des composés à base de gadolinium ; des grappes de bore avec une proportion élevée d'iode ; des polymères, tels que des polysaccharides iodés, des tri-iodobenzènes polymères ; des particules de composés iodés étalant une faible solubilité dans l'eau ; des liposomes contenant des composés iodés ; des lipides iodés, tels que des triglycérides, des acides gras.

13. Procédé selon l'une quelconque des revendications précédentes 1 à 8, dans lequel le matériau qui est utilisé en tant que noyau est sélectionné parmi :
des traceurs à base de ¹¹C, de ¹³N, 13 de ¹⁵O, de ^{66/8}Ga, de ⁶⁰Cu, de ⁵²Fe, de ⁵⁵Co, de ^{61/2/4}Cu, de ^{62/3}Zn, de ^{70/1/4}As, de ^{75/6}Br, de ⁸²Rb, de ⁸⁶Y, de ⁸⁹Zr, de ¹¹⁰In, de ^{120/4}I, de ¹²²Xe et de ¹⁸F, tels que, par exemple, ¹⁸F-FDG (du métabolisme de glucose) ; ¹¹C-méthionine, ¹¹C-tyrosine, ¹⁸F-FMT, ¹⁸F-FMT ou ¹⁸F-FET (des acides aminés) ; ¹⁸F-FMISO, ⁶⁴Cu-ATSM (hypoxie) ; ¹⁸F-FLT, ¹¹C-thymidine, ¹⁸F-FMAU (prolifération).

14. Procédé selon l'une quelconque des revendications précédentes 1 à 8, dans lequel le matériau qui est utilisé en tant que noyau est sélectionné parmi :
des unités d'amélioration de contraste à base de radionucléotides, tels que, par exemple, ^{99m}Tc, ^{123/5/131}I, ⁶⁷Cu, ⁶⁷Ga, ¹¹¹In, ²⁰¹TI.

15. Procédé selon l'une quelconque des revendications précédentes 1 à 5 et 7 à 8, dans lequel le matériau qui est utilisé en tant que noyau est sélectionné parmi :
des toxines, des radio-isotopes, et des chimiothérapiques ; des nanoparticules émettant UV-C, telles que, par exemple, YPO₄:Pr ; des agents de thérapie photodynamique (PDT), tels que, par exemple, des composés à base de structures de porphyrines élargies ; des nucléotides pour la radiothérapie, tels que, par exemple, ¹⁵⁷Sm, ¹⁷⁷Lu, ^{212/3}Bi, ^{186/8}Re, ⁶⁷Cu, ⁹⁰Y, ¹³¹I, ^{114m}In, At, Ra, Ho.

16. Procédé selon l'une quelconque des revendications précédentes 1 à 8, dans lequel le matériau qui est utilisé en tant que noyau est sélectionné parmi :
un transfert de saturation d'échange chimique (CEST) ; des agents de contraste IRM thermosensibles (par exemple, liposomaux) ; des agents de contraste IRM sensibles à pH ; des agents de contraste IRM sensibles à la pression d'oxygène ou aux enzymes ; des agents de contraste IRM dépendant de la concentration d'ions métalliques.

17. Procédé selon l'une quelconque des revendications précédentes 1 à 16, dans lequel le matériau qui est utilisé en tant que noyau est une combinaison de deux ou de plusieurs matériaux.

18. Procédé selon l'une quelconque des revendications précédentes 1 à 5 et 7 à 17, dans lequel le matériau qui est utilisé en tant que coquille(s) est sélectionné parmi :
des acides carboxyliques, des halogénures d'acide, des amines, des anhydrides d'acide, des esters activés, des maléimides, des isothiocyanates, des amines, de l'or, SiO₂, des lipides, des surfactants, un poly-phosphate (par exemple, du poly-phosphate de calcium), un acide aminé (par exemple, de la cystéine), un polymère organique (par exemple, du polyéthylène glycol/PEG, de l'alcool de polyvinyle/PVA, du polyamide, du polyacrylate, de la polyurée), un polymère organique avec des groupes d'extrémité fonctionnels (par exemple, 1,2-distéaroyle-sn-glycéro-3-phosphoéthanolamine-N-[carboxy(polyéthylène glycol)2000] sel d'ammonium), un biopolymère (par exemple, du polysaccharide, tel que du dextran, du xylane, du glycogène, de la pectine, de la cellulose ou du polypeptide, tel que du collagène, de la globuline), de la cystéine ou un peptide avec une teneur élevée en cystéine ou un phospholipide.

19. Procédé selon l'une quelconque des revendications précédentes 1 à 5 et 7 à 17, dans lequel de nouveaux autres composants peuvent être incorporés dans la (les) coquille(s).

20. Procédé selon l'une quelconque des revendications précédentes 1 à 19, dans lequel les polypeptides de l'unité de liaison sont liés chimiquement par le biais de 1 à 3 liants de disulfure à base de cystéine.

21. Procédé selon l'une quelconque des revendications précédentes 1 à 20, dans lequel le matériau qui est utilisé en tant que ligand est sélectionné parmi :
des anticorps (monoclonaux, poly-clonaux, d'une souris, chimériques d'une souris et de l'homme, de l'homme, à chaîne unique, des diacorps, et ainsi de suite), tels que trastuzumab (cancer du sein), rituximab (lymphone non-Hodgkinien), alemtuzumab (leucémie lymphocyte chronique) ; gemtuzumab (leucémie myélogène aiguë) ; edrecolomab (cancer du côlon) ; ibritumomab (lymphome non-Hodgkinien) ; cetuximab (cancer du côlon) ; tositumomab (lymphome non-Hodgkinien) ; epratuzumab (lymphome non-Hodgkinien) ; bevacizumab (cancer du poumon et du côlon) ; anti-CD33 (leucémie myélogène aiguë) ; pemtumomab (cancer de l'ovaire et de l'estomac) ; mittumomab (cancer du poumon et de la peau) ; anti-MUC 1 (adénocarcinome) ; anti-CEA (adénocarcinome) ; anti-CD 64 (des plaques ; des peptides, des polypeptides, des peptidomimétiques, tels que des analogues de somatostatine, des analogues de peptide vasoactif, du neuropeptide Y, des peptides RGD ; des protéines, telles que de l'annexine V, de la protéine activatrice plasminogène tissulaire, des protéines transporteuses ; des macromolécules, par exemple, du hyaluronane, de l'apcitide, du sulfate de dermatane ; des acides nucléiques, tels que des aptamères, des ADN/ARN/ANP anti-sens, des RNA petits interférents ; des lipides, tels que des phospholipides ; des lectines, par exemple, des lectines et des saccharides stimulant les leucocytes.

22. Agents de contraste de ciblage ou agents thérapeutiques de ciblage qui sont produits par un procédé selon l'une quelconques des revendications précédentes 1 à 21.

23. Agents de contraste de ciblage ou agents thérapeutiques de ciblage selon la revendication 22 pour être utilisés dans le diagnostic ou dans la thérapie.

24. Agents de contraste de ciblage ou agents thérapeutiques de ciblage selon l'une quelconque des revendications précédentes 22 à 23 pour être utilisés dans l'imagerie moléculaire de ciblage.

25. Agents de contraste de ciblage selon l'une quelconque des revendications 22 à 23 pour être utilisés dans CT, IRM, TEP, SPECT ou US.

26. Utilisation des agents de contraste de ciblage ou des agents thérapeutiques de ciblage selon l'une quelconque des revendications précédentes 22 à 23 pour la production de composés qui sont appropriés dans le diagnostic ou dans la thérapie.

27. Utilisation des agents de contraste de ciblage ou des agents thérapeutiques de ciblage selon l'une quelconque des revendications précédentes 22 à 23 pour la production de composés qui sont appropriés dans l'imagerie moléculaire de ciblage.

28. Utilisation des agents de contraste de ciblage selon l'une quelconque des revendications précédentes 22 à 23 pour la production de composés qui sont appropriés dans CT, IRM, TEP, SPECT ou US.
